# EUROPEAN PATENT SPECIFICATION

(11) **EP 0 930 853 B1**
(45) Date of publication and mention of the grant of the patent: **23.06.2004**
(21) Application number: 97936275.3
(22) Date of filing: 31.07.1997
(51) Int. Cl.: A61B 19/08, A61B 19/00

(54) **APPARATUS AND METHODS FOR DISPENSING SURGICAL DRAPES**
EINRICHTUNG UND METHODE ZUR ABGABE CHIRURGISCHER ABDECKTÜCHER
APPAREIL ET PROCEDES DE DISTRIBUTION DE CHAMPS OPERATOIRES

(30) Priority: 11.10.1996 US 729442
(43) Date of publication of application: 28.07.1999
(73) Proprietor: MINNESOTA MINING AND MANUFACTURING COMPANY, St. Paul, Minnesota 55133-3427 (US)
(72) Inventor: ASMUS, Robert. A., Saint Paul, MN 55133-3427 (US); BAKER, Dennis, L., Saint Paul, MN 55133-3427 (US); SCHLEI, Dietmar, Saint Paul, MN 55133-3427 (US); SHOR, Rita, M., Saint Paul, MN 55133-3427 (US); THOMAS, Neil, H., Saint Paul, MN 55133-3427 (US)
(74) Representative: VOSSIUS & PARTNER
(86) International application number: PCT/US1997/013295
(87) International publication number: WO 1998/016167

(56) References cited:
- WO-A-95/18046
- GB-A- 1 386 799
- US-A- 4 570 627
- US-A- 5 118 381

## Description

### Field of the Invention

The present invention relates to the field of surgical drapes. More particularly, the present invention provides apparatus and methods for dispensing multiple surgical drapes having a discrete length from a supply of continuous length surgical drape material.

### Background of the Invention

Surgical drapes are used in most surgical procedures to place a barrier between the aseptic operative field and areas that are incapable of surgical cleansing. The drapes also provide a sterile working area on which a physician can place surgical instruments and the like. Surgical drapes are preferably flexible or able to drape such that they follow the contours of the patient's body and drape over the edge of the table on which the patient is located (to avoid interfering with the physician's movement).

Many drapes are disposable to avoid laundering and the associated costs of handling, risk of cross-contamination etc. Disposable drapes may be fluid resistant or impermeable to fluids (in which case they typically incorporate one or more fluid barrier layers such as plastic). They may also resist the passage of fluids containing viral agents and thus may act as viral barriers. The drapes may also be absorbent over the entire surface or portions thereof or they may be non-absorbent over the entire surface or portions thereof. Another feature is that the drapes may exhibit some breathability to water vapor.

Many surgical drapes are provided with specifically designed shapes and openings, commonly referred to as fenestrations, formed therein. In addition the drapes may include pouches for instruments or fluid collection, loops, absorbent pads for fluid collection, and other features. Each drape is typically individually packaged and sterilized or they may be packaged with other items in a kit designed for use in a specific surgical procedure. When each drape is packaged individually, the cost of each drape is increased and the amount of waste generated is also increased due to the packaging materials. The cost to patients is also generally increased because the number of specialty drapes stocked for use in hospitals or surgery centers increases the general overhead charged to patients using the facilities.

In one attempt to address the problem of large inventories of specially designed drapes, a universal surgical draping technique was developed. That technique is described in WO 96/01594 and it typically involves using four individual panels (or sheets) of surgical drape material to square off an incision site. Each panel of the surgical drape material is used to cover a portion of the patient, with the panels arranged in such a manner as to define an opening around a surgical site. The combination of panels used in this method is typically referred to as a universal drape because the panels can be arranged to define an infinite number of openings having different sizes.

Although the use of disposable universal drapes has partially addressed the problem of surgical drape inventories, further reductions would help to lower costs associated with storing and using drapes. More particularly, each of the universal drape panels may be individually packaged and sterilized. As a result, the cost of the universal drapes can be increased due to the additional packaging and the amount of waste generated is also increased by the packaging materials. In addition, because the size of each panel is fixed, a larger inventory of panels having different sizes must be maintained.

In some instances, four panels used to provide a universal drape may be packaged together for use. Although that practice may reduce the packaging cost and waste, it is not without problems. The size of each panel cannot be varied, requiring a larger inventory of packages containing many different combinations of differently sized panels. In many instances, the sizes of the panels packaged together are different and that may result in some confusion on the part of the user as to which panel to apply first and as to where to apply each of the differently-sized panels to create a proper universal drape.

Furthermore, whether packaged individually or together in sets of four, the pre-sized panels used to create universal drapes may also contribute to waste because they are not sized for specific proeedures and, as a result, may be larger than necessary to provide a sterile working area for a given procedure.

As discussed above, whether specially designed surgical drapes are used or panels are provided to form a universal drape, all of the current draping systems for dispensing drapes require inventories and additional packaging that raise the cost of the drapes and can result in increased waste.

US-A-4 570 627 discloses a membrane dispensing assembly comprising a pressure sensitive adhesive membrane and a corresponding underlying coated release paper layer, both being provided with adhesive means. The membrane and paper layer are then formed into and maintained as a substantially cylindrical, rolled configuration which may be dispensed in the form of a plurality of standard fixed lengths.

### Summary of the Invention

The present invention provides systems and methods, for dispensing discrete lengths of surgical drape material from a supply of the same to provide multiple disposable surgical drapes from the supply. Advantages of the present invention include the opportunity to reduce surgical drape inventories and/or packaging material associated with individually packaged drapes. In addition, the ability to provide precisely sized drapes fitted to individual patients may also enhance patient protection from postoperative wound infection.

The present invention provides a surgical drape dispensing system as defined by claim 1.

Preferably the surgical drape dispensing system comprises a dispenser including a blade for separating the discrete lengths of the surgical drape material from the supply of surgical drape material.

Preferably the surgical drape dispensing system comprises surgical drape material with a layer of adhesive on at least a portion of the surgical drape material, and a dispenser including a blade for separating the discrete lengths of the surgical drape material from the supply of surgical drape material.

Preferably the surgical drape dispensing system comprises a plurality of lines of separation in the surgical drape material, each of the plurality of lines being located along the continuous length of the surgical drape material and extending generally transverse to the continuous length.

In yet another aspect, the present invention provides a method for dispensing surgical drape material comprising steps of providing a supply of surgical drape material having a continuous length along which discrete lengths of the surgical drape material are dispensed, and wherein the drape material in the roll includes at least one fold extending along the continuous length of the drape material, and dispensing a discrete length of the surgical drape material by separating the discrete length of surgical drape material from the supply of surgical drape material. The method may include a step of placing the supply in a dispenser if desired.

These and other features and advantages of some illustrative dispensing systems and methods according to the present invention are set forth in more detail below.

### Brief Description of the Drawing

Figure 1 is a perspective view of one supply of surgical drape material in roll form.
Figure 2 is an end view of another supply of surgical drape material not falling under the scope of the appended claims.
Figure 3 is a schematic cross-sectional diagram of the surgical drape material of Figure 1 taken across line 3-3.
Figure 4 is a plan view of a discrete length of surgical drape material separated from the supply, the surgical drape material including indicia indicating length and adhesive along one edge thereof.
Figure 5 is a schematic cross-sectional view of a roll of surgical drape material in a housing including a blade.
Figure 6 is a perspective view of the dispensing system of Figure 5 in an opened pouch.
Figure 7 is a perspective view of an alternate dispensing system.
Figure 8 is a plan view of a portion of surgical drape material depicting a plurality of lines of perforations in the surgical drape material.
Figure 9 is a perspective view of one enclosure for dispensing surgical drape material.
Figure 10 is a schematic diagram of one method of dispensing surgical drape material.
Figure 11 is an enlarged cross-sectional view of a composite including surgical drape material located between two liners.
Figure 12 is a schematic block diagram of one system for dispensing surgical drape material.

### Detailed Description of the Invention

The present invention provides systems, including apparatus and methods, for dispensing discrete lengths of surgical drape material from a supply of the same to provide disposable surgical drapes. The surgical drape material may have any combination of features such as breathability, fluid resistance, fluid impermeability, resistance to migration of viral agents, impermeability to migration of viral agents, absorbency, etc. The present invention may also be used to dispense incise drapes. Regardless of the features exhibited by the surgical drape materials used in connection with the present invention, the surgical drape material can be dispensed economically and conveniently. Examples of some suitable drape materials and adhesives that may be used in surgical drapes are described in, for example, WO 96/01594.

The present invention is particularly advantageous when used to provide surgical drapes for use in the universal draping technique as described above. By use of the present invention, drapes of any desired length may be dispensed from a larger supply, thereby reducing the inventory problems associated with individually packaged, pre-sized universal drape panels. The present invention may also be useful in reducing packaging material costs and waste, particularly when the universal drape panels are packaged individually. Furthermore, the size of the universal drape panels dispensed according to the present invention can be precisely controlled by the user.

Although the invention may find particular advantages when used to dispense universal surgical drape panels, it will be understood that it may also be used to dispense specialty drapes with various sized openings or fenestrations to provide access to a surgical site. It may also be used to dispense incises drapes or specialty drapes including incise drape material in the fenestrations or with special pockets or pouches attached to the surface of the drape.

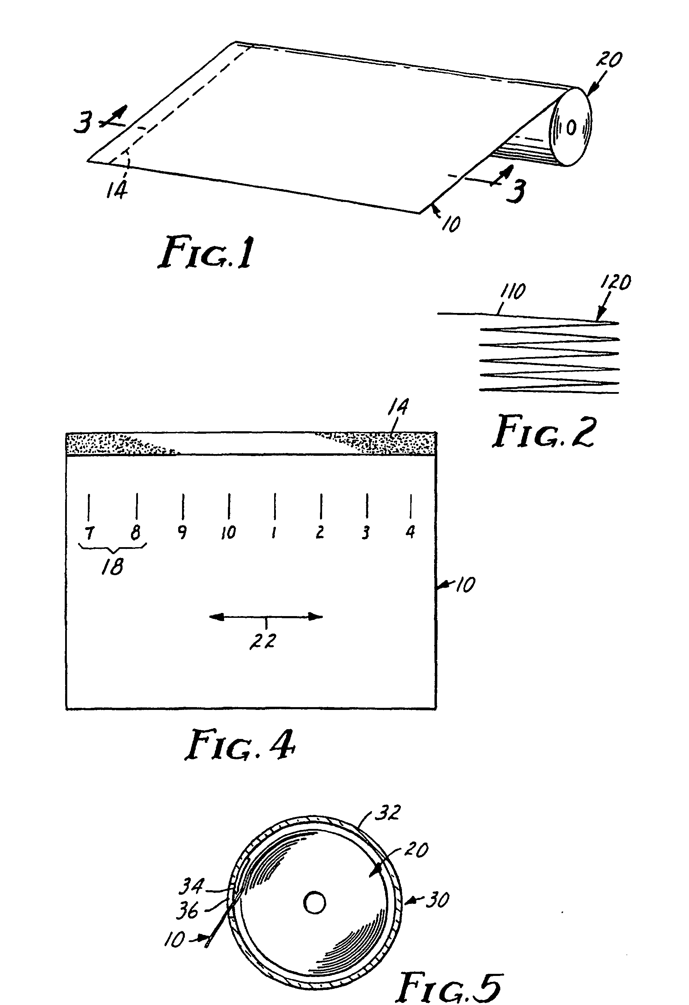

Figure 1 depicts one supply of surgical drape material 10 comprising a supply 20 from which the drape material 10 is dispensed in discrete lengths as determined by the user.

The supply 20 of surgical drape material is provided with a continuous length along which the material is dispensed in discrete lengths. The continuous length provided in supply 20 is preferably long enough to allow dispensing of multiple discrete lengths of the surgical drape material 10 from the supply 20. By dispensing surgical drape material 10 from a supply 20 having a continuous length sufficient to supply multiple drapes having discrete lengths, the problems and costs associated with individually packaged surgical drapes can be avoided or reduced. The supply 20 may be used to provide multiple drapes for use with a single patient, or, alternatively, the supply 20 may be used to provide one or more surgical drapes to each of two or more patients.

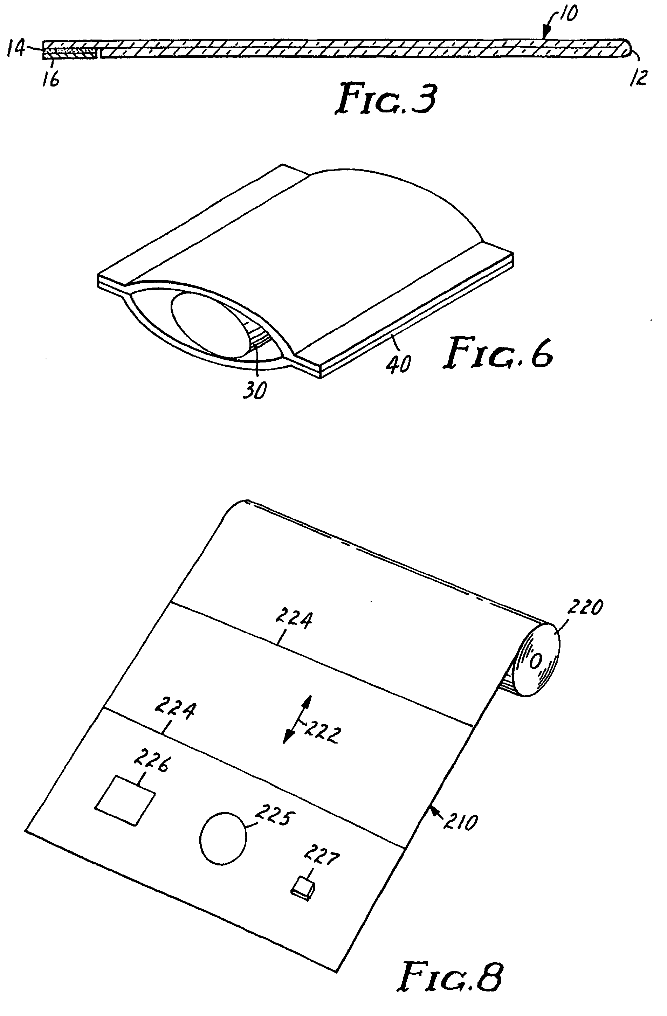

The surgical drape material 10 will typically, but not necessarily, have a fixed width. The term fixed width will be understood to include embodiments in which the surgical drape material 10 includes one or more folds 12 running along the continuous length of the material 10 when dispensed as seen in Figure 3 (a cross-sectional view of material 10 in Figure 1 along line 3-3). As a result, after dispensing the material 10, it may be unfolded to a desired width for use. The use of one or more folds 12 can help to equalize the thickness of the drape material 10 that is not coated with an adhesive 14 and optional liner 16 to facilitate storing the material 10 on a roll 20. Folding can also reduce the length of the roll 20, i.e., the width of the drape material 10 as dispensed.

Turning to Figure 4, a discrete length of surgical drape material 10 is depicted after being separated from, for example, supply 20. If the drape material 10 is designed for use in universal draping, it will typically include adhesive 14 along one edge as shown. The adhesive used can be any suitable adhesive, although it will typically comprise a pressure-sensitive adhesive that forms a barrier to migration of foreign matter (including, preferably, bacterial and viral agents) when applied to the skin of a patient. It is also preferable that the adhesive 14 be biocompatible to reduce skin irritation.

It will be understood that if the drape material 10 is intended to be used for incise draping applications, it may include adhesive over a majority of the surface of the drape material 10, with the edges of the drape material 10 typically being free from adhesive to assisting application of the drape material 10 to a patient.

Figure 4 also depicts another optional feature of the present invention, i.e., indicia 18 indicative of the length of the surgical drape material 10 dispensed from the supply. The indicia 18 may be printed on the drape material 10 or embossed into the material 10. It may include numbers or may comprise a series of marks spaced along the length of the drape material 10.

Turning now to Figure 5, one system 30 for dispensing surgical drape material 10 according to the present invention is depicted. The system 30 includes a supply 20 of drape material 10 and a dispenser 32. In many instances it will be preferred that the dispenser 32 at least partially enclose the roll 20, but that is not required. The dispenser 32 shown in Figure 5 includes a blade 34 attached proximate the opening 36 in the dispenser 32 through which the drape material 10 is dispensed. The blade 34 may have a straight edge, although it is preferably serrated to facilitate clean separation of the drape material 10 into the desired discrete lengths.

The dispenser 32 may substantially enclose the roll 20 as shown or alternatively, it may provide more open, skeletal support for the roll and an optional blade. Examples of the variations in dispensing systems useful for dispensing surgical drape material in accordance with the present invention can be seen in the variety of dispensers designed for packaging tapes, SCOTCH™ Brand transparent tapes, cellophane tapes, etc. One example of a suitable dispenser can be found in WO 96/11870, titled DISPENSING ASSEMBLY.

To dispense the drape material 10 using dispenser 32, a user grasps the material 10 and pulls it from the supply 20 within the dispenser 32. When the desired length of drape material has been removed, the user applies force to the drape material 10 with the blade 34 to separate or cut the discrete length of drape material 10 from the supply 20.

A dispenser 32 as shown in Figure 5 may be used as a disposable device or, alternatively, it may be reusable with the supply 20 of surgical drape material being replaced when empty. If reusable, the dispenser 32 may be sterilized between changes in the supply 20 of surgical drape material 10 to assist in sterile delivery of the drape material 10.

If the dispenser 32 is disposable, the dispenser 32 and associated supply 20 of surgical drape material 10 may be packaged for sterile delivery at the site at which the drape material 10 will be dispensed. One example of a suitable package depicted in Figure 6 is a pouch 40 of any suitable flexible materials such as TYVEK™ (a spun-bonded olefin sheet material available from E.I. DuPont de Nemours, Inc., Wilmington, Delaware), plastic, plastic and metallic foil laminates, etc., that may be heat sealed or otherwise closed with the dispensing system 30 inside. The combination of surgical drape material dispensing system 30 and pouch 40 can then be sterilized as a unit using any suitable technique such as gamma radiation or ethylene oxide. Such packaging methods will be well known to those skilled in the art.

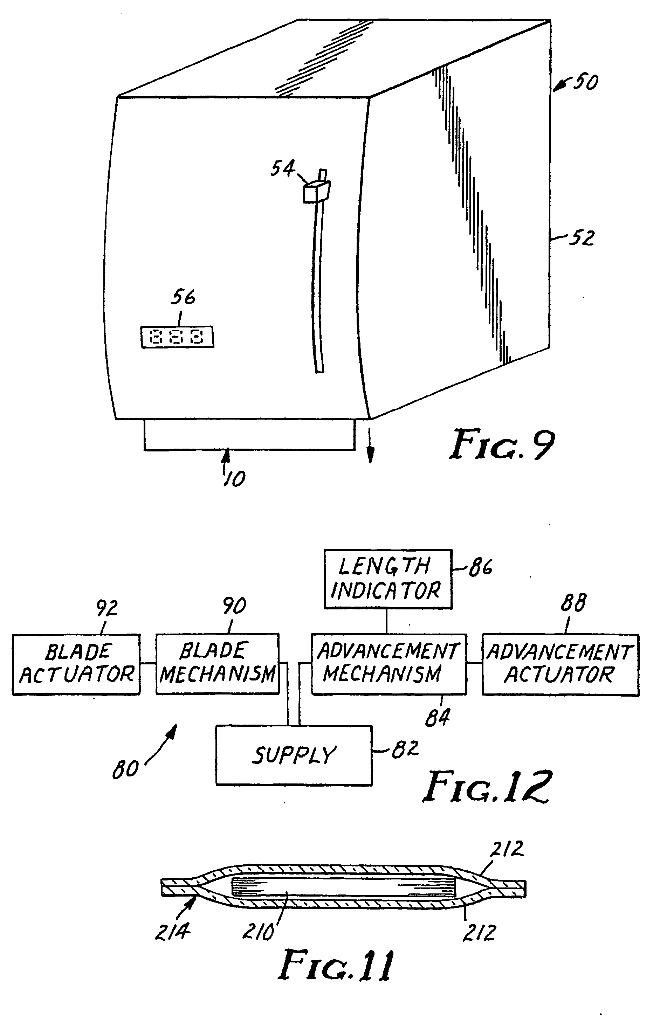

In one alternative, a system 130 depicted in Figure 7 includes a dispenser 132 that functions as part of the packaging for the roll of surgical drape material (not shown). As depicted in Figure 7, the dispenser 132 includes an opening 136 that is sealed by a removable cover 138. The dispenser body 132 and cover 138 combine to form a sealed volume in which the supply of sterile surgical drape material can be maintained sterile until the package is opened, i.e., the cover 138 is removed. The cover 138 can be provided in the form of, for example, an adhesive tape or any suitable material or construction that can seal the opening 136 from penetration that could compromise sterility of the supply of surgical drape material before dispensing.

In another variation, it will be understood that a supply 20 of surgical drape material may also be packaged alone, i.e., without a dispenser, in a disposable pouch or other suitable packaging to maintain sterility of the surgical drape material 10 until use. If the supply 20 of surgical drape material 10 is supplied alone, i.e., without a dispenser, it may be dispensed from the supply 20 to the desired length and cut into discrete lengths using a pair of scissors or other sharp instrument. If the surgical drape material 10 includes lines of separation such as perforations or a scored line as described more fully below in connection with Figure 8, the discrete lengths of the surgical drape material can be separated from the supply roll by applying the proper amount of tension to the drape material 10 after dispensing the desired length from the supply 20.

In another variation, the supply 20 of surgical drape material may also be supplied with surgical instruments and other supplies in a kit or pack for use in specific surgical procedures. If so provided, separate packaging of the supply 20 of surgical drape material may be unnecessary because the kit or pack will typically be packaged and sterilized as a unit.

Where the supply 20 of surgical drape material is supplied as a single-use supply for universal draping, i.e., use in preparation for one procedure after which any remaining universal drape material in supply 20 would be discarded, the length of universal drape material 10 provided in supply 20 would preferably be about 4 feet (1.2 meters) to about 30 feet (9 meters). More preferably, a single-use roll 20 of universal drape material would contain about 6 feet (1.8 meters) to about 20 feet (6 meters) of surgical drape material 10. If the supply 20 were intended to be used with more than one patient (typically in a dispenser) the continuous length of the surgical drape material would preferably be about 9 meters or more, more preferably about 15 meters or more.

Figure 8 depicts a roll 220 of surgical drape material 210 according to the present invention in which a plurality of lines of separation 224 are provided. The lines 224 preferably extend across the width of the drape material 210 (generally perpendicular to the length of the material 210 as represented by arrow 222). The lines 224 are also preferably evenly spaced, but may be variably spaced if desired. Each line 224 may comprise a series of perforations through one or more layers of the drape material 210 or, alternately, each line may be a scored lined embossed into one or more layers of the drape material 210. In either case, the lines 224 facilitate separation of discrete lengths of drape material 210 from the supply roll 220 along the line 224.

Supply roll 220 in which the material 210 includes lines of separation 224 may be provided alone, in combination with a dispenser such as, for example, dispenser 32 as depicted in Figure 5, or any other dispensing system as described herein. Typically, however, the need for a blade or other cutting device will be limited or non-existent due to the lines of separation supplied in the drape material 210.

The supply roll 220 of drape material 210 with lines of separation 224 may find particular application when used to supply surgical drapes that are not used in the universal draping technique. For example, one or more fenestrations 225, one or more pouches 226, one or more absorbent pads 227, etc., could be provided on the surface of the drape material 210 in the proper location with respect to the lines of separation 224.

Turning now to Figure 9, an alternate dispensing system 50 is depicted that is similar in many respects to paper towel dispensers. The system 50 includes a dispenser 52 containing a supply of surgical drape material 10 (not shown, but typically in roll form). The dispenser 52 includes a lever 54 that actuates an advancement mechanism (not shown) within the dispenser 52 to advance the drape material 10 out of the dispenser 52 for dispensing. The dispenser 52 may include a blade or other mechanism for separating discrete lengths of the drape material 10 from the supply within the dispenser 52. In addition or alternatively, the drape material 10 may include lines of separation as described above with respect to Figure 8.

Although the dispenser 52 is depicted as including a lever 54 for actuating the advancement mechanism, it will be understood that a rotating knob or lever could alternatively be supplied for advancing the drape material 10 from the dispenser 52.

Also depicted as a part of system 50 is an optional indicator 56 that provides information to a user as to the length of drape material 10 being dispensed from the dispenser 52. The indicator 56 could be, for example, mechanical, electrical or electro-mechanical in nature and could also provide an indication when the supply of drape material 10 in the dispenser 52 was nearing exhaustion. A simple indicator could include a window in dispenser 52 to allow visual observation of the supply of surgical drape material.

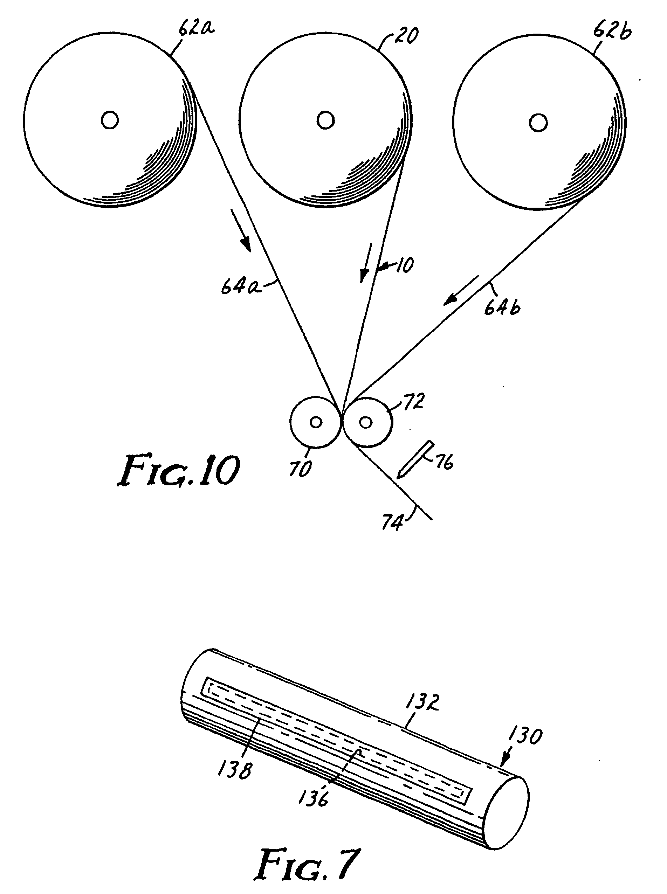

One potential concern with dispensing surgical drapes from a reusable dispenser such as dispenser 52 is contamination of the drape material during dispensing by the mechanisms used to deliver the drape material 10. One attempt to address that concern is depicted in Figure 10, a schematic diagram of one method for sterile dispensing of drape material 10 from a reusable dispenser such as dispenser 52.

As shown in Figure 10, a supply roll 20 of surgical drape material would be provided. The drape material 10 would be threaded through the nip formed by rolls 70 and 72. Two rolls 62a and 62b of liner material 64a and 64b (referred to generally as 64) would also be provided and threaded through the nip formed by rolls 70 and 72. As a result, the composite web 74 comprising surgical drape material 10 in between two layers of liner 64 would exit from the nip rolls 70 and 72 where the composite web 74 could be separated into discrete lengths by, for example, blade 76.

The liner materials 64 could be provided of any suitable material that would prevent contact between the drape material 10 and the rolls 70 and 72. The liner materials 64 could include release coatings to facilitate separation from any adhesives on the drape material 10. Additionally, the liners 64 could be wider than the drape material 10 and sealed along the edges to form a sleeve in which the drape material 10 would be located. The sealing between liners 64 could be accomplished by heat sealing, ultrasonic welding, cohesive materials, or any other suitable material and/or method.

As an alternative to laminating the surgical drape material 10 in between liners 64 at the delivery site, it may also be helpful to provide the drape material pre-laminated in between two liners. Figure 11 is an enlarged cross-sectional view of one such delivery system in which a layer of surgical drape material 210 is located between two liners 212 to form a composite web 214. The view in Figure 11 is along the continuous length of the supply of surgical drape material 210. As shown the liners 212 may be wider than the surgical drape material 210. As a result, the liners 212 may be bonded or otherwise sealed to each other outside of the drape material 210 as discussed above with respect to liners 64 to provide a sleeve in which the drape material 210 is dispensed. As a result, if a supply of the composite web 214 was provided in, for example, dispensers such as 32 or 52 described above, the drape material could be dispensed in a manner-in which only the ends of the drape material 210 contact anything before use.

Because of the wide variations in the exact design of apparatus for dispensing surgical drape material in accordance with the present invention, Figure 12 is a schematic block diagram indicating one possible embodiment of the system of the present invention. The system 80 includes a supply 82 of surgical drape material in any format, e.g., roll or folded stack.

The system 80 may include an advancement mechanism 84 for advancing the supply of drape material for dispensing. Examples of advancement mechanisms includes the mechanisms used for paper towel dispensers, i.e., rotating levers or knobs, ratcheting levers or any other suitable mechanism. Alternatively, the advancement may be accomplished by having the operator pull on the drape material to remove it from the supply 82. Automated systems may include motors (e.g., stepper, alternating current, direct current, air, etc.) or other power sources to reduce physical interaction of the user with the dispensing system 80.

As indicated with respect to dispensing system 50 above, the advancement mechanism 84 may be coupled to an indicator 86 for displaying the amount of drape material being dispensed and/or indicating when the supply 82 is running low. The indicator 86 may be electrical, mechanical, or electro-mechanical in nature as desired.

The advancement mechanism 84 may be coupled to an advancement actuator 88, particularly in systems that are more automated. The advancement actuator 88 may include a lever 54 as depicted in Figure 8, or it could include a more complicated combination of mechanical, electrical, and/or electro-mechanical components needed to actuate the advancement mechanism 84. Such combinations will be well known to those skilled in the art of designing such systems.

One advancement actuator 88 could be designed for foot activation by a user to reduce the potential for contaminating the user's hands when dispensing the drape material. The foot activated system could include a pedal or pressure pad in combination with other components to activate the advancement mechanism 84. Another contemplated advancement actuator 88 would be a non-contact advancement actuator, i.e., the user would not be required to physically contact any structure to initiate the advancement mechanism 84. One example of a non-contact advancement actuator 88 could include a proximity sensor, such as a photocell, that would activate the advancement mechanism 84 when, for example, a user's hands are near the supply 82.

Also depicted in Figure 12 is a blade mechanism 90 of any design suitable to cut or separate the drape material into discrete lengths. In some embodiments of system 80, the blade mechanism 90 could be as simple as a straight edge over which a perforated or scored drape material can be easily separated. In other embodiments, it could include a fixed sharpened blade with either a straight or serrated edge, a rolling circular blade, a cutting wire, etc. In more automated systems, the blade mechanism 90 could include a moving blade that shears the drape material in, e.g., a scissoring action. In other systems, the blade mechanism 90 could include a blade, such as an angled or circular blade, that is moved across the width of the drape material by a motor, piston, etc. to separate discrete lengths from the supply 82.

The blade mechanism 90 could be moved manually or it could be automated. When automated, the system 80 would include a blade actuator 92 to initiate movement of the blade mechanism 90 to effectuate separation of the drape material. The blade actuator 92 could include any combination of mechanical, electrical, and/or electro-mechanical components needed to actuate the blade mechanism 90. Such combinations will be well known to those skilled in the art of designing such systems.

One blade actuator 92 could be designed for foot activation by a user to reduce the potential for contaminating the user's hands when dispensing the drape material. The foot activated system could include a pedal or pressure pad in combination with other components to activate the blade mechanism 90. Another contemplated blade actuator 92 would be a non-contact blade actuator, i.e., the user would not be required to physically contact any structure to initiate the blade mechanism 90. One example of a non-contact blade actuator 92 could include a proximity sensor, such as a photocell, that would activate the blade mechanism 90 when, for example, a user's hands are near a specified location.

The above-described apparatus for dispensing surgical drape material can be used according to the method of the present invention. That method includes the steps of providing a supply of surgical drape material having a width and a continuous length along which the surgical drape material is dispensed and dispensing a discrete length of the surgical drape material by separating the discrete length of surgical drape material from the supply of surgical drape material. It is preferred, but not required, that the surgical drape material be supplied sterile.

It is also preferred that the supply of surgical drape material be provided near the site at which the discrete lengths of drape material will be used. For example, the supply of drape material could be located within an operating room or other location where the drape material would be used as preparation for a surgical procedure or other medical procedure requiring surgical drape material.

The step of separating discrete lengths of the surgical drape material from the supply can be performed using one or more blades or the drape material can be provided with lines of separation, e.g., perforations or score lines, to facilitate separation of discrete lengths from the larger supply of drape material.

Methods according to the present invention may also include steps of advancing the discrete lengths of surgical drape material from a dispenser containing the supply and, optionally, moving a blade relative to the drape material or moving the drape material relative to fixed blade to effectuate separation of the discrete length of drape material from the supply. The advancing of drape material and/or moving of the blade can be accomplished manually or through actuators. If actuators are used, it is preferred that they be foot operated or operated using some other method in which the user's hands are not required to touch the dispenser to reduce the chance for contamination of a user's hands.

The patents, patent documents, and publications cited herein are incorporated by reference in their entirety, as if each were individually incorporated by reference. Various modifications and alterations of this invention will become apparent to those skilled in the art without departing from the scope of the invention, and it should be understood that this invention is achieved by the features of the claims and any obvious modifications thereof.

## Claims

1. A system of dispensing surgical drape material comprising a roll (20, 220, 82) of surgical drape material (10; 210) having a continuous length along which the surgical drape material is dispensable in a plurality of discrete lengths as determined by a user, **characterised in that** the drape material in the roll includes at least one fold (12) extending along the continuous length of the drape material, whereby the width of the roll is less than the width of the drape material when unfolded.

2. A system according to claim 1 wherein the roll is located in a disposable package (40).

3. A system of dispensing surgical drape material according to claim 1, further comprising:
a dispenser (32; 132; 50) including a blade (34) for separating the discrete lengths of the surgical drape material from the supply of surgical drape material.

4. A system according to claim 3 wherein the dispenser (132) further comprises a removable cover (138) over an opening (136) in the dispenser, and further wherein the dispenser and cover enclose the supply of surgical drape material.

5. A system of dispensing surgical drape material according to claim 1, wherein the roll (220) of surgical drape material (210) includes a plurality of lines of separation (224) in the surgical drape material, each of the plurality of lines being located along the continuous length of the surgical drape material and extending generally transverse to the continuous length.

6. A system according to claim 1 or 5 wherein the surgical drape material is located between two liners (64a, 64b) extending along the continuous length of the surgical drape material.

7. A system according to claim 6 wherein the liners are wider than the surgical drape material and further wherein the liners are sealed to each other at their edges to form a sleeve in which the surgical drape material is located.

8. A system according to claim 1 or 5 further comprising a layer of adhesive on at least a portion of the surgical drape material.

9. A system according to claim 5 further comprising a dispenser in combination with the supply of surgical drape material.

10. A system according to claim 3 or 9 wherein the dispenser further comprises an advancement mechanism (84) for advancing a discrete length of the surgical drapo material from the dispenser.

11. A method for dispensing surgical drape material utilizing a system according to claim 1, comprising the step of providing a roll of surgical drape material having a continuous length along which the surgical drape material is dispensable in a plurality of discrete lengths as determined by a user, wherein the drape material in the roll includes at least one fold extending along the continuous length of the drape material, whereby the width of the roll is less than the width of the drape material when unfolded, and the step of dispensing a discrete length of the surgical drape material by separating the discrete length of surgical drape material from the roll of surgical drape material.

12. A system according to claim 1 wherein the surgical drape material has first and second edges extending along its continuous length, a layer of adhesive (14) at least substantially adjacent the first edge of the surgical drape material, a liner (16) covering the layer of adhesive, and wherein the surgical drape material is folded so that no portion of the drape material along its continuous length is underneath the layer of adhesive.

## Patentansprüche

1. System zur Abgabe chirurgischen Abdeckmaterials, umfassend eine Rolle (20, 220, 82) mit chirurgischem Abdeckmaterial (10, 210) von kontinuierlicher Länge, über die das chirurgische Abdeckmaterial in mehreren, von einem Anwender festgelegten diskreten Längen abgegeben werden kann, **dadurch gekennzeichnet, dass** das Abdeckmaterial in der Rolle zumindest eine Falte (12) enthält, die über die kontinuierliche Länge des Abdeckmaterials verläuft, wodurch die Breite der Rolle geringer als die Breite des Abdeckmaterials im ungefalteten Zustand ist.

2. System nach Anspruch 1, bei dem die Rolle in einer Einwegpackung (40) angeordnet ist.

3. System zur Abgabe chirurgischen Abdeckmaterials nach Anspruch 1, ferner umfassend: einen Spender (32, 132, 50) mit einer Klinge (34) zum Abtrennen der diskreten Längen des chirurgischen Abdeckmaterials von dem Vorrat an chirurgischem Abdeckmaterial.

4. System nach Anspruch 3, worin der Spender (132) ferner eine abnehmbare Abdeckung (138) über einer Öffnung (136) im Spender umfasst und der Spender und die Abdeckung den Vorrat an chirurgischem Material umschließen.

5. System zur Abgabe von chirurgischem Abdeckmaterial nach Anspruch 1, bei dem die Rolle (220) mit chirurgischem Abdeckmaterial (210) mehrere Trennlinien (224) im chirurgischen Abdeckmaterial enthält, wobei jede der mehreren Linien über die kontinuierliche Länge des chirurgischen Abdeckmaterials verläuft und sich im Allgemeinen quer zur kontinuierlichen Länge erstreckt.

6. System nach Anspruch 1 oder 5, bei dem das chirurgische Abdeckmaterial zwischen zwei Zwischenlagen (64a, 64b) angeordnet ist, die über die kontinuierliche Länge des chirurgischen Abdeckmaterials verlaufen.

7. System nach Anspruch 6, bei dem die Zwischenlagen breiter sind als das chirurgische Abdeckmaterial und ferner die Zwischenlagen an ihren Rändern aneinander befestigt sind, um eine Hülle zu formen, in der sich das chirurgische Abdeckmaterial befindet.

8. System nach Anspruch 1 oder 5, ferner umfassend eine Schicht aus Klebstoff auf zumindest einem Teil des chirurgischen Abdeckmaterials.

9. System nach Anspruch 5, ferner umfassend einen Spender in Kombination mit dem Vorrat an chirurgischem Abdeckmaterial.

10. System nach Anspruch 3 oder 9, bei dem der Spender ferner einen Vorschiebemechanismus (84) zum Vorschieben einer diskreten Länge des chirurgischen Abdeckmaterials aus dem Spender umfasst.

11. Verfahren zur Abgabe eines chirurgischen Abdeckmaterials unter Verwendung eines Systems nach Anspruch 1, umfassend die Schritte der Bereitstellung einer Rolle mit chirurgischem Abdeckmaterial von kontinuierlicher Länge, über die das chirurgische Abdeckmaterial in mehreren, von einem Anwender festgelegten diskreten Längen abgegeben werden kann, wobei das Abdeckmaterial in der Rolle zumindest eine Falte enthält, die über die kontinuierliche Länge des Abdeckmaterials verläuft, wodurch die Breite der Rolle geringer als die Breite des Abdeckmaterials im ungefalteten Zustand ist, und der Abgabe einer diskreten Länge des chirurgischen Abdeckmaterials durch Abtrennen der diskreten Länge des chirurgischen Abdeckmaterials von der Rolle mit chirurgischem Abdeckmaterial.

12. System nach Anspruch 1, bei dem das chirurgische Abdeckmaterial einen ersten und einen zweiten Rand, die über seine kontinuierliche Länge verlaufen, eine Schicht aus Klebstoff (14) zumindest im Wesentlichen neben dem ersten Rand des chirurgischen Abdeckmaterials und eine Zwischenlage (16), die die Klebstoffschicht verdeckt, enthält und so gefaltet ist, dass kein Teil des Abdeckmaterials über seine kontinuierliche Länge unter der Klebstoffschicht liegt.

## Revendications

1. Système de distribution de tissu de champ opératoire, comprenant un rouleau (20, 220, 82) de tissu de champ opératoire (10, 210) ayant une longueur continue le long de laquelle le tissu de champ opératoire peut être distribué dans une pluralité de longueurs discrètes déterminées par un utilisateur, **caractérisé en ce que** le tissu de champ présent dans le rouleau comprend au moins un pli (12) qui s'étend le long de la longueur continue du tissu de champ, la largeur du rouleau étant inférieure à la largeur du tissu de champ quand celui-ci est déplié.

2. Système selon la revendication 1., dans lequel le rouleau se trouve dans un emballage jetable (40).

3. Système de distribution de tissu de champ opératoire selon la revendication 1, comprenant en outre :
un distributeur (32, 132, 50) comportant une lame (34) pour la séparation des longueurs discrètes du tissu de champ opératoire à partir du tissu de champ opératoire fourni.

4. Système selon la revendication 3, dans lequel le distributeur (132) comprend en outre une pièce de recouvrement amovible (138) sur une ouverture (136) du distributeur, et en outre dans lequel le distributeur et la pièce de recouvrement entourent le tissu de champ opératoire fourni.

5. Système de distribution de tissu de champ opératoire selon la revendication 1, dans lequel le rouleau (220) de tissu de champ opératoire (210) comporte une pluralité de lignes de séparation (224) dans le tissu de champ opératoire, chacune de la pluralité de lignes se trouvant le long de la longueur continue du tissu de champ opératoire et s'étendant généralement en sens transversal par rapport à la longueur continue.

6. Système selon la revendication 1 ou 5, dans lequel le tissu de champ opératoire se trouve entre deux doublures (64a, 64b) qui s'étendent le long de la longueur continue du tissu de champ opératoire.

7. Système selon la revendication 6, dans lequel les doublures sont plus larges que le tissu de champ opératoire, et en outre dans lequel les doublures sont scellées l'une à l'autre sur leurs bords pour former un manchon dans lequel se trouve le tissu de champ opératoire.

8. Système selon la revendication 1 ou 5, comprenant en outre une couche d'adhésif sur au moins une partie du tissu de champ opératoire.

9. Système selon la revendication 5, comprenant en outre un distributeur combiné avec le tissu de champ opératoire fourni.

10. Système selon la revendication 3 ou 9, dans lequel le distributeur comprend en outre un mécanisme d'avancement (84) pour l'avancement d'une longueur discrète du tissu de champ opératoire à partir du distributeur.

11. Méthode de distribution de tissu de champ opératoire utilisant un système selon la revendication 1, comprenant l'étape qui consiste à fournir un rouleau de tissu de champ opératoire ayant une longueur continue le long de laquelle le tissu de champ opératoire peut être distribué dans une pluralité de longueurs discrètes déterminées par un utilisateur, dans lequel le tissu de champ dans le rouleau comprend au moins un pli qui s'étend le long de la longueur continue du tissu de champ, la largeur du rouleau étant inférieure à la largeur du tissu de champ quand celui-ci est déplié, et l'étape qui consiste à distribuer une longueur discrète du tissu de champ opératoire en séparant la longueur discrète de tissu de champ opératoire du rouleau de tissu de champ opératoire.

12. Système selon la revendication 1, dans lequel le tissu de champ opératoire comporte un premier bord et un deuxième bord qui s'étendent le long de sa longueur continue, une couche d'adhésif (14) au moins fondamentalement adjacente au premier bord du tissu de champ opératoire, et une doublure (16) recouvrant la couche d'adhésif, et dans lequel le champ opératoire est plié de sorte qu'aucune partie du tissu de champ le long de sa longueur continue ne se trouve sous la couche d'adhésif.
